(19) Europäisches Patentamt
European Patent Office
Office européen des brevets

(11) EP 2 295 027 A2

(12) # EUROPÄISCHE PATENTANMELDUNG

(43) Veröffentlichungstag:
**16.03.2011 Patentblatt 2011/11**

(21) Anmeldenummer: **10168392.8**

(22) Anmeldetag: **05.07.2010**

(51) Int Cl.:
***A61K 8/44*** *(2006.01)* ***A61K 8/46*** *(2006.01)*
***A61K 8/49*** *(2006.01)* ***A61Q 5/10*** *(2006.01)*
***D06P 3/14*** *(2006.01)*

(84) Benannte Vertragsstaaten:
**AL AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**BA ME RS**

(30) Priorität: **31.08.2009 DE 102009029043**

(71) Anmelder: **Henkel AG & Co. KGaA**
**40589 Düsseldorf (DE)**

(72) Erfinder:
- **Metten, Diane**
  **40229, Düsseldorf (DE)**
- **Weser, Gabriele**
  **41472, Neuss (DE)**
- **Boßmann, Britta**
  **40699, Erkrath (DE)**

(54) **Intensive, schonende Färbemittel**

(57) Die vorliegende Anmeldung betrifft kosmetische Mittel zur oxidativen Färbung keratinischer Fasern, insbesondere menschlicher Haare, enthaltend in einem kosmetischen Träger mindestens eine farbverändernde Komponente, mindestens ein von Luftsauerstoff verschiedenes Oxidationsmittel und mindestens ein Acetylpyridiniumderivat der Formel (I),

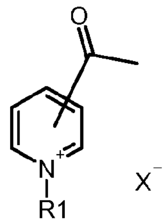

(I),

wobei dass das Mittel frei von Ammoniak ist.

EP 2 295 027 A2

## Beschreibung

Die vorliegende Erfindung betrifft Mittel zur oxidativen Färbung keratinischer Fasern, insbesondere menschlichen Haaren, enthaltend kationische Acetylpyridiniumderivate, eine farbverändernde Komponente und Wasserstoffperoxid, wobei die Mittel frei von Ammoniak sind, sowie deren Verwendung zur Intensivierung der Färbeleistung und Verbesserung der Aufhellung.

Die Veränderung von Form und Farbe der Haare stellt einen wichtigen Bereich der modernen Kosmetik dar. Zur modischen Farbgestaltung von Frisuren oder zur Kaschierung von ergrautem oder gar weißem Haar mit modischen oder natürlichen Farbtönen greift der Verbraucher zu farbverändernden Mitteln. Diese Mittel sollen neben der gewünschten Färbeleistung möglichst minimale Schädigungen auf dem Haar hervorrufen und die Faserstruktur schonen. Um die Schädigung weiterhin zu reduzieren, ist es notwendig, dass die Mittel durch Zusätze in ihrer Färbeleistung möglichst verbessert werden, so dass intensivere und lang anhaltende Farben erzielt werden, ohne die Mengen an Färbemittel zu erhöhen.

Zur Bereitstellung farbverändernder kosmetischer Mittel, insbesondere für keratinhaltige Fasern, wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich zwar durch hervorragende, lang anhaltende Färbeergebnisse aus. Für natürlich wirkende Färbungen muss aber üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen.

Eine weitere Möglichkeit zur Farbveränderung bietet die Verwendung von Färbemitteln, die sogenannte Oxofarbstoffvorprodukte, die sich in zwei Klassen unterteilen, enthalten. Eine erste Klasse der Oxofarbstoffvorprodukte sind Verbindungen mit mindestens einer reaktiven Carbonylgruppe. Eine zweite Klasse der Oxofarbstoffvorprodukte bilden C,H-acide Verbindungen und Verbindungen mit primärer oder sekundärer Aminogruppe oder Hydroxygruppe, vorzugsweise aromatischen Verbindungen. Die vorgenannten Komponenten sind im Allgemeinen selbst keine Farbstoffe, und eignen sich daher jede für sich genommen allein nicht zur Färbung keratinhaltiger Fasern. Die resultierenden Färbungen besitzen teilweise Farbechtheiten auf der keratinhaltigen Faser, die mit denen der Oxidationsfärbung vergleichbar sind, ohne jedoch auf Oxidationsmittel zur Farbstoffausbildung angewiesen zu sein. Bei einem weiteren Färbeverfahren werden Vorstufen des natürlichen Haarfarbstoffes Melanin auf das Haar aufgebracht, die im Rahmen oxidativer Prozesse im Haar naturanaloge Farbstoffe aus bilden. Die Ausfärbung kann dabei mit Luftsauerstoff als einzigem Oxidationsmittel erfolgen, so dass auf keine weiteren Oxidationsmittel zurückgegriffen werden muss.

Insbesondere oxidative Haarfärbemittel sind trotz ihrer vorteilhaften Färbeeigenschaften für den Anwender mit Nachteilen behaftet. Vor ihrer Anwendung auf menschliches Haar werden oxidative Färbemittel üblicherweise mit verdünnter wässriger Wasserstoffperoxid-Lösung vermischt. Diese Mischung wird dann auf das Haar aufgebracht und nach einer bestimmten Einwirkzeit wieder ausgespült. Der Einsatz dieser Oxidationsmittel zur Ausfärbung respektive Entwicklung der eigentlichen Färbung führt zu Schädigungen in der Haarstruktur und auf der Haaroberfläche. Eine Verbesserung der Färbeleistung eines Mittels hinsichtlich Farbintensität würde eine Reduktion von Anwendungsmenge oder Anwendungsdauer erlauben.

Oxidative Färbeprozesse an keratinischen Fasern laufen üblicherweise bei alkalischen pH-Werten ab, insbesondere zwischen 9,0 und 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Als Alkalisierungsmittel wird üblicherweise Ammoniak eingesetzt, der allerdings für den Anwender den Nachteil des intensiven Geruches und eventueller Reizung bis hin zu Hautirritationen und Hautsensibilisierungen aufweist.

Auch wenn die bislang auf dem Markt befindlichen Färbemittel in der Regel gute Färbeleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden.

Aufgabe der vorliegenden Erfindung ist es daher, die Intensität und Leuchtkraft der Färbungen von ammoniakfreien Haarfärbemitteln soweit zu verbessern, dass sie mit den üblichen, sich auf dem Markt befindlichen, ammoniakhaltigen Mitteln vergleichbar oder ihnen überlegen sind, gleichzeitig jedoch eine verringerte Haarschädigung aufweisen.

Es wurde nun in nicht vorhersehbarer Weise gefunden, dass durch Zusatz bestimmter kationischen Acylpyridiniumderivaten zu farbverändernden, oxidativen Mitteln eine signifikante Verbesserung der Farbintensität bei ammoniakarmenFärbungen keratinischer Fasern erzielt werden kann.

Der Einsatz von kationischen Acylpyridiniumderivaten in der Haarfärbung ist beispielsweise aus den Schriften DE 10148845 A1 oder DE 10261656 A1 bekannt. In beiden Dokumenten werden diese Derivate jedoch zusammen mit mindestens einer zweiten färbenden Komponente als Mittel zur oxidationsmittelfreien Färbung beschrieben. Aus dem Stand der Technik ist bislang nicht ersichtlich, dass Acylpyridiniumderivate in spezieller Kombination mit Wasserstoffperoxid in ammoniakfreien Färbemitteln unter Erhalt hervorragender Färbeleistungen eingesetzt werden können.

Ein erster Gegenstand der vorliegenden Erfindung ist daher ein Mittel zur oxidativen Färbung keratinischer Fasern, insbesondere menschlicher Haare, enthaltend in einem kosmetischen Träger mindestens eine farbverändernde Komponente, mindestens ein von Luftsauerstoff verschiedenes Oxidationsmittel und mindestens ein Acetylpyridiniumderivat der Formel (I),

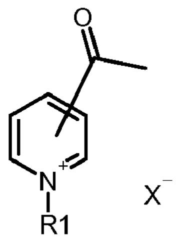

(I),

worin

- R1 für eine $C_1$-$C_6$-Alkylgruppe, eine $C_2$-$C_6$-Alkenylgruppe, eine $C_2$-$C_6$-Hydroxyalkylgruppe, eine $C_1$-$C_6$-Alkoxy-$C_2$-$C_6$-alkylgruppe, eine Carboxy-$C_2$-$C_6$-alkylgruppe, eine Aryl-$C_1$- $C_6$-alkylgruppe, eine Heteroaryl-$C_1$-$C_6$-alkylgruppe, eine Arylgruppe oder eine Heteroarylgruppe und
- $X^-$ für ein physiologisch verträgliches Anion stehen,

welches dadurch gekennzeichnet ist, dass das Mittel frei von Ammoniak ist.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die zur erfindungsgemäßen Verwendung eingesetzten Zubereitungen enthalten die Wirkstoffe in einem kosmetischen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Die Begriffe "frei von Ammoniak" bzw. "ammoniakarm" und "ammoniakfrei" im Sinne der Erfindung beziehen sich dabei auf die dem Mittel zugesetzte Ammoniakmenge, wobei der Ammoniakzusatz sowohl als wässrige, alkoholische, wässrig-alkoholische oder anderweitige Lösung als auch durch Ammoniakgaseinleitung oder Zusatz von verflüssigtem Ammoniak erfolgen kann. Der Zusatz von Ammoniak kann aber auch durch Verwendung von entsprechenden Ammonium-Salzen erfolgen, wobei das Ammonium-Kation je nach pH-Wert der Zubereitung im Gleichgewicht mit seiner korrespondierenden Base, dem Ammoniak selbst, steht. Die Begriffe "ammoniakfrei" bzw. "ammoniakarm" im Sinne der Erfindung beziehen sich daher auch auf Mittel, die Ammonium-Salze enthalten.

Der Begriff "frei von Ammoniak" bedeutet dabei, dass das anwendungsbereite Mittel weniger als 2 Gew.-% zugesetzten Ammoniak, bezogen auf das Gesamtgewicht der Anwendungszubereitung, enthält. Sofern Ammonium-Salze im anwendungsbereite Mittel vorhanden sind, so beträgt der Ammoniakanteil, der sich aus diesen Salzen unter Annahme einer vollständigen Deprotonierung der Ammonium-Kationen ergibt, entsprechend weniger als 2 Gew.-%, bezogen auf das Gesamtgewicht der Anwendungszubereitung. Bevorzugte, ammoniakarme Mittel enthalten weniger als 1 Gew.-%, insbesondere weniger als 0,5 Gew.-% und ganz besonders bevorzugt weniger als 0,1 Gew.-% zugesetzten Ammoniak, jeweils bezogen auf das Gesamtgewicht der Anwendungszubereitung. Ammoniakfrei im Sinne der Erfindung

sind solche Mittel, denen kein Ammoniak durch eine der oben beschriebenen Methoden zugesetzt wurde. Solche Mittel sind erfindungsgemäß besonders bevorzugt.

Als ersten wesentlichen Inhaltsstoff enthält die Färbezubereitung mindestens ein Acetylpyridiniumderivat der Formel (I).

Beispiele für die als Substituenten der Verbindungen der Formel (I) genannten Reste sind:

Beispiele für $C_1$-$C_6$-Alkylreste sind die Gruppen $-CH_3$, $-CH_2CH_3$, $-CH_2CH_2CH_3$, $-CH(CH_3)_2$, $-CH_2CH_2CH_2CH_3$, $-CH_2CH(CH_3)_2$, $-CH(CH_3)CH_2CH_3$, $-C(CH_3)_3$.

Beispiele für eine $C_2$-$C_6$-Alkenylgruppe sind eine Prop-2-enylgruppe (Allylgruppe), eine 2-Methyl-prop-2-enylgruppe, eine But-3-enylgruppe, eine But-2-enylgruppe, eine Pent-4-enylgruppe oder eine Pent-3-enylgruppe, wobei die Prop-2-enylgruppe bevorzugt ist.

Beispiele für eine $C_2$-$C_6$-Hydroxyalkylgruppe sind $-CH_2CH_2OH$, $-CH_2CH_2CH_2OH$, $-CH_2CH(OH)CH_3$ und $-CH_2CH_2CH_2CH_2OH$, wobei die Gruppe $-CH_2CH_2OH$ bevorzugt ist.

Beispiele für $C_1$-$C_6$-Alkoxy-$C_2$-$C_6$-alkylgruppen sind die Gruppen $-CH_2CH_2OCH_3$, $-CH_2CH_2CH_2OCH_3$, $-CH_2CH_2OCH_2CH_3$, $-CH_2CH_2CH_2OCH_2CH_3$, $-CH_2CH_2OCH(CH_3)_2$, $-CH_2CH_2CH_2OCH(CH_3)_2$.

Beispiele für eine Carboxy-$C_1$-$C_6$-alkylgruppe sind die Carboxymethylgruppe, die 2-Carboxyethylgruppe oder die 3-Carboxypropylgruppe.

Beispiele für Aryl-$C_1$-$C_6$-alkylgruppen sind die Benzylgruppe und die 2-Phenylethylgruppe.

Beispiele für eine Heteroaryl-$C_1$-$C_6$-alkylgruppe sind die Pyridin-2-ylmethylgruppe, die Pyridin-3-ylmethylgruppe, die Pyridin-4-ylmethylgruppe, die Pyrimidin-2-ylmethylgruppe, die Pyrrol-1-ylmethylgruppe, die Pyrrol-1-ylethylgruppe, die Pyrazol-1-ylmethylgruppe oder die Pyrazol-1-ylethylgruppe.

Beispiele für eine Arylgruppe sind die Phenylgruppe, die 1-Naphthylgruppe oder die 2-Naphthylgruppe.

Beispiele für eine Heteroarylgruppe sind die Pyridin-2-ylgruppe, die Pyridin-3-ylgruppe, die Pyridin-4-ylgruppe, die Pyrimidin-2-ylgruppe, die Pyrrol-1-ylgruppe, die Pyrrol-2-ylgruppe, die Pyrazol-1-ylgruppe, die Pyrazol-3-ylgruppe oder die Pyrazol-4-ylgruppe.

In einer Ausführungsform der vorliegenden Erfindung sind solche Verbindungen gemäß Formel (I) bevorzugt, bei welchen der Rest R1 der allgemeinen Struktur (I) für eine $C_1$-$C_6$-Alkylgruppe, für eine $C_2$-$C_6$-Alkenylgruppe oder für eine $C_2$-$C_6$-Hydroxyalkylgruppe steht. Es ist erfindungsgemäß bevorzugt, wenn der Rest R1 für eine $C_1$-$C_6$-Alkylgruppe, bevorzugt für Methyl, Ethyl, n-Propyl oder Isopropyl und insbesondere bevorzugt für Methyl, steht.

Es ist bevorzugt, wenn das Anion $X^-$ gemäß Formel (I) ausgewählt wird aus Halogenid, insbesondere Chlorid, Bromid und Iodid, Benzolsulfonat, p-Toluolsulfonat, $C_1$-$C_4$-Alkylsulfonat, Trifluormethansulfonat, Acetat, Triflluoracetat, Perchlorat, ½ Sulfat, Hydrogensulfat, Tetrafluorborat, Hexafluorphosphat oder Tetrachlorzinkat. Erfindungsgemäß besonders begünstigt ist es, wenn das physiologisch verträgliche Anion $X^-$ für ein Halogenidion (insbesondere Chlorid oder Bromid), Hydrogensulfat, ½ Sulfat, p-Toluolsulfonat, Benzolsulfonat oder Acetat steht.

Es hat sich herausgestellt, dass die Acetylpyridiniumderivate gemäß Formel (I) erfindungsgemäß besonders vorteilhafte Eigenschaften besitzen, wenn sie die Acetyl-Gruppe entweder in 2- oder 4-Position am Pyridin-Ring tragen. Eine weitere Ausführungsform der vorliegenden Erfindung ist **dadurch gekennzeichnet, dass** das Mittel als Acetylpyridiniumderivat gemäß Formel (I) mindestens ein 2-Acetylpyridiniumderivat und/oder 4-Acetylpyridiniumderivat enthält.

Geeignete Acetylpyridiniumderivate sind dabei die physiologisch verträglichen Salze, die als Kation ein Acetylpyridiniumderivat, ausgewählt aus 4-Acetyl-1-methylpyridinium, 4-Acetyl-1-allylpyridinium, 4-Acetyl-1-(2-hydroxyethyl)pyridinium, 2-Acetyl-1-methylpyridinium, 2-Acetyl-1-allylpyridinium und 2-Acetyl-1-(2-hydroxyethyl)pyridinium, enthalten.

Insbesondere sind solche Mittel erfindungsgemäß bevorzugt, die dadurch gekennzeichnet sind, dass das Acetylpyridiniumderivat gemäß Formel (I) ausgewählt ist aus der Gruppe, die gebildet wird aus 4-Acetyl-1-methylpyridinium-p-toluolsulfonat, 4-Acetyl-1-methylpyridinium-benzolsulfonat, 4-Acetyl-1-methylpyridiniumbromid, 4-Acetyl-1-methylpyridiniumchlorid, 4-Acetyl-1-methylpyridiniumhydrogensulfat, 4-Acetyl-1-methylpyridiniumacetat, 4-Acetyl-1-allylpyridinium-p-toluolsulfonat, 4-Acetyl-1-allylpyridinium-benzolsulfonat, 4-Acetyl-1-allylpyridiniumbromid, 4-Acetyl-1-allylpyridiniumchlorid, 4-Acetyl-1-allylpyridiniumhydrogensulfat, 4-Acetyl-1-allylpyridiniumacetat, 2-Acetyl-1-methylpyridinium-p-toluolsulfonat, 2-Acetyl-1-methylpyridinium-benzolsulfonat, 2-Acetyl-1-methylpyridiniumbromid, 2-Acetyl-1-methylpyridiniumchlorid, 2-Acetyl-1-methylpyridiniumhydrogensulfat, 2-Acetyl-1-methylpyridiniumacetat, 2-Acetyl-1-allylpyridinium-p-toluolsulfonat, 2-Acetyl-1-allylpyridinium-benzolsulfonat, 2-Acetyl-1-allylpyridiniumbromid, 2-Acetyl-1-allylpyridiniumchlorid, 2-Acetyl-1-allylpyridiniumhydrogensulfat und/oder 2-Acetyl-1-allylpyridiniumacetat.

Erfindungsgemäß bevorzugte Mittel sind **dadurch gekennzeichnet, dass** sie als das Acetylpyridiniumderivat gemäß Formel (I) eine Verbindung, ausgewählt aus 4-Acetyl-1-methylpyridinium-p-toluolsulfonat und/oder 2-Acetyl-1-methylpyridinium-p-toluolsulfonat, insbesondere 4-Acetyl-1-methylpyridinium-p-toluolsulfonat, enthält.

In einer Ausführungsform enthalten die erfindungsgemäßen Färbemittel die Acetylpyridiniumderivate der Formel (I) zu 00,01 bis 5 Gew.-%, bevorzugt von 0,05 bis 3 Gew.-% und insbesondere von 0,1 bis 2 Gew.-%, jeweils

bezogen auf das Gesamtgewicht des anwendungsbereiten Färbemittels, enthält.

Als weiteren wesentlichen Inhaltsstoff enthalten die erfindungsgemäßen Färbemittel mindestens eine farbverändernde Komponente.

Die farbverändernde Komponente wird dabei ausgewählt aus mindestens einem Oxidationsfarbstoffvorprodukt und/oder direktziehenden Farbstoff und/oder naturanalogen Farbstoff.

In einer Ausführungsform der vorliegenden Erfindung enthält das Mittel als farbverändernde Komponente mindestens ein Oxidationsfarbstoffvorprodukt und/oder direktziehenden Farbstoff. In einer bevorzugten Ausführungsform der erfindungsgemäßen Verwendung enthält die Färbezubereitung als farbverändernde Komponente mindestens ein Oxidationsfarbstoffvorprodukt.

Als Oxidationsfarbstoffvorprodukte enthalten die Färbezubereitungen mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)-propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)-phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze. Die Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Erfindungsgemäße Kupplerkomponenten werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Dibeziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetrahydrochinoxalin, und/oder Gemischen aus zwei oder mehreren Verbindungen aus einer oder mehreren dieser Klassen.

Erfindungsgemäß bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis(2,4-diaminophenyl)-propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)-ethanol, 2-({3-[(2-Hydroxyethyl)amino]-4,5-dimethylphenyl}amino)ethanol, 2-[3-Morpholin-4-yl-phenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Weiterhin können die Mittel zur erfindungsgemäßen Verwendung mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen.

Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl) amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt.

Besonders geeignet als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und/oder 5,6-Dihydroxyindolin-2-carbonsäure, insbesondere bevorzugt 5,6-Dihydroxyindolin. Als Vorstufen naturanaloger Haarfarbstoffe hervorragend geeignet sind weiterhin Derivate des 5,6-Dihydroxyindols , insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere bevorzugt 5,6-Dihydroxyindol.

Als dritten wesentlichen Inhaltsstoff enthält das Mittel ein als von Luftsauerstoff verschiedenes Oxidationsmittel. Hierzu können prinzipiell alle zur kosmetischen Anwendung geeigneten Oxidationsmittel eingesetzt werden. Hierzu zählen Wasserstoffperoxid, Persulfatsalze, Peroxid-Salze, jedoch ebenso geeignete Enzyme.

Eine bevorzugte Ausführungsform der vorliegenden Erfindung ist jedoch **dadurch gekennzeichnet, dass** das Mittel als von Luftsauerstoff verschiedenes Oxidationsmittel mindestens ein Oxidationsmittel, ausgewählt aus Wasserstoffperoxid und seinen Anlagerungsverbindungen an feste anorganische oder organische Verbindungen, enthält. Bevorzugt wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidon·n $H_2O_2$ (n ist eine positive ganze Zahl größer 0), Harnstoffperoxid und Melaminperoxid, eingesetzt werden. I

m letztgenannten Fall setzen die Anlagerungsverbindungen in der erfindungsgemäßen Anwendungsmischung Wasserstoffperoxid frei, d. h. diese Mittel enthalten neben der Anlagerungsverbindung im kosmetischen Träger freies Wasserstoffperoxid.

Erfindungsgemäß ganz besonders bevorzugt wird das Wasserstoffperoxid dem erfindungsgemäßen Mittel als wässrige Wasserstoffperoxid-Lösung zudosiert. Die Konzentration einer Wasserstoffperoxid-Lösung wird einerseits von den gesetzlichen Vorgaben und andererseits von dem gewünschten Effekt bestimmt; vorzugsweise werden 6 bis 12 gew.-%ige Lösungen in Wasser verwendet. Erfindungsgemäß bevorzugte Mittel sind **dadurch gekennzeichnet, dass** sie - bezogen auf ihr Gesamtgewicht - 0,01 bis 12 Gew.-%, vorzugsweise 0,1 bis 10 Gew.-%, besonders bevorzugt 1 bis 6 Gew.-% Wasserstoffperoxid (berechnet als 100%iges $H_2O_2$) enthalten.

Unter Berücksichtigung der bisher genannten bevorzugten Ausführungsformen stellt es eine spezielle und ausdrücklich bevorzugte Ausführungsform dar, wenn das Mittel zum Färben von keratinischen Fasern frei von Ammoniak ist und in einem kosmetischen Träger als erste Komponente mindestens eine Verbindung ausgewählt aus der Gruppe, die gebildet wird aus 4-Acetyl-1-methylpyridinium-p-toluolsulfonat und 2-Acetyl-1-methylpyridinium-p-toluolsulfonat, weiterhin mindestens ein Oxidationsfarbstoffprodukt, ausgewählt aus p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salzen, und als dritte Komponente Wasserstoffperoxid in den bereits beschriebenen bevorzugten Mengenanteilen enthält.

Ganz besonders bevorzugt sind schließlich Mittel, die die nachfolgende Kombination enthalten, worin sich die Gewichtsangaben wiederum auf das Gesamtgewicht des anwendungsbereiten Mittels beziehen:

0,1 bis 4,0 Gew.-% 4-Acetyl-1-methylpyridinium-p-toluolsulfonat,
0,1 bis 5,0 Gew.-% an Oxidationsfarbstoffvorprodukten und
0,1 bis 12,0 Gew.-% Wasserstoffperoxid.

Es hat im Laufe der Untersuchungen dieser Erfindung herausgestellt, dass der Zusatz bestimmter anionischer Tenside eine weitere Verbesserung der Färbeleistung hinsichtlich Aufhellvermögen, Intensität und Farbigkeit bewirken kann.

Eine weitere Ausführungsform der vorliegenden Erfindung ist daher **dadurch gekennzeichnet, dass** das Mittel zusätzlich mindestens eine Verbindung ausgewählt aus der Gruppe, die gebildet wird aus N-Acylsarcosin-Derivaten der Formel (II) und N-Acyltaurin-Derivaten der Formel (III),

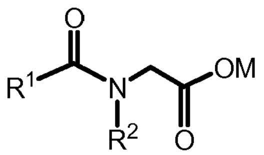
(II),
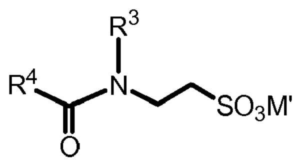
(III),

worin

| | |
|---|---|
| $R^1$ und $R^4$ | jeweils unabhängig voneinander für eine $C_7$-$C_{30}$-Alkylgruppe, $C_7$-$C_{30}$-Alkenylgruppe oder eine $C_7$-$C_{30}$-Hydroxyalkylgruppe stehen, |
| $R^2$ | für eine $C_1$-$C_4$-Alkylgruppe oder eine $C_1$-$C_4$-Hydroxyalkylgruppe steht, |
| $R^3$ | für ein Wasserstoffatom, eine $C_1$-$C_4$-Alkylgruppe oder eine $C_1$-$C_4$-Hydroxy- alkylgruppe steht und |
| M und M’ | jeweils voneinander unabhängig für ein Wasserstoffatom oder ein Äquivalent eines ein- oder mehrwertigen Kations stehen, |

enthält.

Die Reste $R^1$ der Formel (II) und $R^4$ der Formel (III) stehen bevorzugt unabhängig voneinander für eine $C_9$-$C_{23}$-Alkylgruppe, $C_9$-$C_{23}$-Alkenylgruppe oder eine $C_9$-$C_{23}$-Hydroxyalkylgruppe, besonders bevorzugt unabhängig voneinander für einen Rest, ausgewählt aus der Liste, die gebildet wird, aus Nonyl, Undecyl, Tridecyl, Pentadecyl, Heptadecyl, Nonadecyl, Henicosanyl, 15-Methylhexadecyl, Heptadec-8-enyl, Heptadeca-8,11-dienyl, Nonadeca-4,7,10,13-tetraenyl, Heptadeca-8,11,14-trienyl und 11-Hydroxyheptadecyl.

Der Rest $R^2$ gemäß Formel (II) steht bevorzugt für einen Rest, ausgewählt aus der Gruppe, die gebildet wird aus Methyl, Ethyl, Isopropyl, n-Propyl und 2-Hydroxyethyl. Besonders bevorzugt steht der Rest $R^2$ für eine Methylgruppe. Der Rest $R^3$ gemäß Formel (III) steht bevorzugt für einen Rest, ausgewählt aus der Gruppe, die gebildet wird aus Wasserstoffatom, Methyl, Ethyl, Isopropyl, n-Propyl und 2-Hydroxyethyl, besonders bevorzugt für ein Wasserstoffatom oder eine Methylgruppe. Es ist erfindungsgemäß ganz besonders bevorzugt, wenn $R^2$ der Formel (II) und $R^3$ der Formel (III) beide für eine Methylgruppe stehen.

Wenn die Verbindungen der Formeln (II) bzw. (III) als Säure vorliegen, bedeutet der Rest M bzw. der Rest M' ein Wasserstoffatom. Wenn die Verbindungen der Formeln (II) bzw. (III) als Salz vorliegen, stehen M bzw. M bzw. M' steht in den Formeln (II) bzw. (III) bevorzugt für ein Wasserstoffatom, ein Ammoniumion, ein Alkalimetallion, für ein halbes Äquivalent eines Erdalkalimetallions oder ein halbes Äquivalent eines Zinkions, besonders bevorzugt für ein Wasserstoffatom, ein Ammoniumion, ein Natriumion, ein Kaliumion, ½ Calciumion, ½ Magnesiumion oder ½ Zinkion.

Die Verbindungen der Formel (II) werden bevorzugt ausgewählt aus mindestens einem Vertreter aus der Gruppe, die gebildet wird, aus N-Lauroylsarcosin, N-Myristoylsarcosin, N-Palmitoylsarcosin, N-Oleylsarcosin, N-Cocoylsarcosin (Cocoyl entspricht der Zusammensetzung des Fettsäureschnitt des Kokosnussöls) und N-Palmkernsarcosin (Palmkern entspricht der Zusammensetzung des Fettsäureschnitt des Palmkernöls), sowie aus den Salzen der vorgenannten N-Acylsarcosinderivate.

Die Verbindungen der Formel (III) werden bevorzugt ausgewählt aus mindestens einem Vertreter aus der Gruppe, die gebildet wird, aus N-Lauroyl-N-methyltaurin, N-Stearoyl-N-methyltaurin, N-Palmitoyl-N-methyltaurin, N-Myristoyl-N-methyltaurin, N-Oleyl-N-methyltaurin, N-Cocoyl-N-methyltaurin, N-Palmkern-N-methyltaurin, sowie aus den Salzen dieser N-Acyltaurinderivate.

Die Wirkstoffe der Formel (II) und der Formel (III) werden bevorzugt in Kombination, d.h. mindestens eine Verbindung gemäß Formel (II) gemeinsam mit mindestens einer Verbindung gemäß Formel (III), verwendet. Eine Ausführungsform der vorliegenden Erfindung ist daher **dadurch gekennzeichnet, dass** das Mittel zusätzlich mindestens eine Verbindung, ausgewählt aus N-Acylsarcosin-Derivaten der Formel (II), und mindestens eine Verbindung, ausgewählt aus N-Acyltaurin-Derivaten der Formel (III), enthält.

Besonders bevorzugt enthält das erfindungsgemäße Mittel zusätzlich Natrium Myristoylsarcosinat als N-Acylsarcosin-Derivaten der Formel (II) und Natrium N-Methyl-cocoyltaurat N-Acyltaurin-Derivaten der Formel (III). Eine solche Kombination wird beispielsweise von der Firma Zschimmer & Schwarz unter dem Handelsnamen Protelan MST 35 vertrieben.

Die Verbindungen der Formel (II) und die Verbindungen der Formel (III) werden, wenn sie gemeinsam zum Einsatz kommen, bevorzugt in einem Gewichtsverhältnisbereich von 1 zu 3 bis 3 zu 1, besonders bevorzugt von 1.5 zu 1 bis 1 zu 1.5, ganz besonders bevorzugt von 1 zu 1, verwendet.

Die erfindungsgemäßen Mittel enthalten die Verbindungen der Formel (II) und/oder der Formel (III) bevorzugt in einer Menge von 0,1 bis 15 Gew.-%, besonders bevorzugt von 0,5 bis 10 Gew.-%, ganz besonders bevorzugt von 0,5 bis 8 Gew.-%, jeweils bezogen auf das Gewicht des anwendungsbereiten Farbveränderungsmittels.

Weiterhin hat sich der Zusatz bestimmter weiterer Wirkstoffe als erfindungsgemäß besonders geeignet herausgestellt. Zu diesen Wirkstoffen zählen Aminosäuren und Xanthine, insbesondere Taurin (2-Aminoethansulfonsäure), Glycin, L-Serin, L-/R-Serin und/oder Coffein. Eine weitere Ausführungsform der vorliegenden Erfindung ist **dadurch gekennzeichnet, dass** das Mittel zusätzlich mindestens eine Verbindung, ausgewählt aus der Gruppe, die gebildet wird aus Taurin (2-Aminoethansulfonsäure), Glycin, L-Serin, L-/R-Serin und/oder Coffein, enthält.

Die erfindungsgemäßen Mittel enthalten die Verbindungen, ausgewählt aus Taurin, Glycin, L-Serin, L-/R-Serin und/oder Coffein, bevorzugt in einer Menge von 0,01 bis 15 Gew.-%, besonders bevorzugt von 0,05 bis 10 Gew.-%, ganz besonders bevorzugt von 0,1 bis 5 jeweils bezogen auf das Gewicht des anwendungsbereiten Mittels.

Erfindungsgemäß kann ein Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Weiterhin hat es sich als vorteilhaft erweisen, wenn die Färbemittel mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Bei einer Anwendung von zusätzlichen Oxidationsmitteln wird die eigentliche Färbezubereitung zweckmäßigerweise unmittelbar vor der Anwendung durch Mischung einer erfindungsgemäßen Zubereitung, enthaltend mindestens

eine farbverändernde Komponente und mindestens eine Aminosäure, sowie einer Zubereitung, enthaltend das zusätzliche Oxidationsmittel, insbesondere Wasserstoffperoxid, hergestellt.

Die erfindungsgemäß verwendbaren Mittel können zusätzlich Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet. Für die starke Aufhellung sehr dunklen Haares ist jedoch der alleinige Einsatz von Wasserstoffperoxid oder dessen Anlagerungsprodukten an organische beziehungsweise anorganische Verbindungen oftmals nicht ausreichend.

Daher kann es erfindungsgemäß, sollte der Verbraucher den Wunsch nach einer sehr starken Blondierung verspüren, in einer weiteren Ausführungsform bevorzugt sein, wenn das Färbemittel zusätzlich mindestens ein anorganisches Persulfatsalz bzw. Peroxodisulfatsalz in dem Mittel zum Aufhellen der keratinischen Fasern enthält. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Ferner können die erfindungsgemäßen Mittel weitere Wirk-, Hilfs- und Zusatzstoffe enthalten, wie beispielsweise kationische Polymere, nichtionische Polymere; zwitterionische und amphotere Polymere; anionische Polymere; Verdickungsmittel; haarkonditionierende Verbindungen; Proteinhydrolysate pflanzlicher oder tierischer Herkunft; Parfümöle, Dimethylisosorbid und Cyclodextrine; faserstrukturverbessernde Wirkstoffe; Entschäumer; Farbstoffe; Antischuppenwirkstoffe; Lichtschutzmittel; Wirkstoffe; Vitamine, Provitamine und Vitaminvorstufen, insbesondere solche der Gruppen A, $B_3$, $B_5$, $B_6$, C, E, F und H; Pflanzenextrakte; pflanzliche Öle; Cholesterin; Konsistenzgeber; Fette und Wachse (Fettalkohole, Bienenwachs, Montanwachs und Paraffine); Quell- und Penetrationsstoffe; Trübungsmittel; Perlglanzmittel; Treibmittel; Antioxidantien.

Die Auswahl dieser weiteren Stoffe wird der Fachmann gemäß den gewünschten Eigenschaften der Zubereitungen treffen. Die erfindungsgemäß eingesetzten Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die Färbezubereitungen der erfindungsgemäßen Verwendung weisen bevorzugt einen pH-Wert im Bereich von 4 bis 12 aufweist. Im Fall von Oxidationsfärbemitteln findet die Anwendung der Färbemittel in einem schwach alkalischen Milieu statt, bevorzugt bei einem pH-Wert im Bereich von 8,0 bis 10,5. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann neben dem möglichst zu vermeidenden Ammoniak gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen, basische Aminosäuren und Alkanolaminen. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, organische Alkalisierungsmittel werden bevorzugt ausgewählt aus Alkanolaminen aus primären, sekundären oder tertiären Aminen mit einem $C_2$-$C_6$-Alkylgrundkörper, der mindestens eine Hydroxylgruppe trägt. Besonders bevorzugte Alkanolamine werden ausgewählt aus 2-Aminoethan-1-ol (Monoethanolamin), 2-Amino-2-methylpropan-I-ol, 2-Amino-2-methyl-propan-1,3-diol und Triethanolamin. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Besonders bevorzugt sind Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin. Bevorzugt werden Acidificierungsmittel und Alkalisierungsmittel jeweils in einer Menge von 0,05 bis 15 Gew.-%, insbesondere von 0,5 bis 10 Gew.-%, bezogen auf das Gesamtgewicht des anwendungsbereiten Mittels, eingesetzt.

Das anwendungsbereite Färbemittel wird auf die keratinischen Fasern aufgetragen und für eine bestimmte Einwirkzeit auf der Faser, insbesondere im Haar belassen. Die Auftragung der Färbezubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex (Einweghandschuhe). Es ist aber auch möglich, die Färbezubereitung mit einer Applikationshilfe auf die keratinischen Fasern aufzutragen. Die Auftragungs- und die Einwirktemperatur der Färbezubereitung beträgt Raumtemperatur bis 45°C. Gegebenenfalls kann die Wirkung der Färbezubereitung durch externe Wärmezufuhr, wie beispielsweise mittels einer Wärmehaube, verstärkt werden. Die bevorzugte Einwirkdauer der Färbezubereitung auf die keratinische Faser beträgt 2 bis 45 min, bevorzugt 5 bis 35 min. Nach

Beendigung der Einwirkdauer wird das verbliebene Färbemittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet.

Ein weiterer Gegenstand der vorliegenden Erfindung ist die kosmetische, nicht therapeutische Verwendung eines Mittels des ersten Erfindungsgegenstands zur Intensivierung der Färbeleistung bei der oxidativen Färbung keratinischer Fasern, insbesondere menschlicher Haare.

Ein weiterer Gegenstand der vorliegenden Erfindung ist die kosmetische, nicht therapeutische Verwendung eines Mittels des ersten Erfindungsgegenstands zur Verbesserung der Aufhellleistung und/oder Farbigkeit bei der oxidativen Färbung keratinischer Fasern, insbesondere menschlicher Haare.

Die erfindungsgemäßen Mittel können direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden.

Ein weiterer Gegenstand der vorliegenden Erfindung ist eine Mehrkomponentenverpackungseinheit (Kit-of-Parts),
umfassend mindestens einem ersten Container (C1) mit einer Färbezubereitung (A), enthaltend in einem kosmetischen Träger

a) mindestens ein Oxidationsfarbstoffvorprodukt und/oder direktziehenden Farbstoff und
b) mindestens ein Acetylpyridiniumderivat der Formel (I),

und mindestens einen zweiten Container (C2) mit einer Entwicklerzubereitung (B), enthaltend in einem kosmetischen Träger mindestens ein Oxidationsmittel,
welche dadurch gekennzeichnet ist, dass sowohl Färbezubereitung (A) als auch Entwicklerzubereitung (B) frei von Ammoniak sind.

Der Begriff "Container" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff "Container" umfasst daher, ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Komponenten der Färbezubereitung können in einem einzelnen Container enthalten sein, es ist aber auch möglich und gegebenenfalls bevorzugt, sie auf verschiedene Behälter aufzuteilen und den Verbraucher anzuleiten, sie vor der Anwendung miteinander zu mischen.

Eine bevorzugte Ausführungsform des Erfindungsgegenstands ist eine Mehrkomponentenverpackungseinheit, welche dadurch gekennzeichnet ist, dass die Färbezubereitung (A) zusätzlich mindestens eine Verbindung ausgewählt aus der Gruppe, die gebildet wird aus N-Acylsarcosin-Derivaten der Formel (II) und N-Acyltaurin-Derivaten der Formel (III), enthält.

Eine weitere bevorzugte Ausführungsform dieses Erfindungsgegenstands ist eine Mehrkomponentenverpackungseinheit, welche dadurch gekennzeichnet ist, dass die Färbezubereitung (A) zusätzlich mindestens eine Verbindung, ausgewählt aus N-Acylsarcosin-Derivaten der Formel (II), und mindestens eine Verbindung, ausgewählt aus N-Acyltaurin-Derivaten der Formel (III), enthält.

In einer besonders bevorzugten Ausführungsform ist die Verpackungseinheit dadurch gekennzeichnet, dass sie mindestens eine zusätzliche Komponente, ausgewählt aus persönlicher Schutzbekleidung, wie Einweghandschuhen, Schürze, Applikationshilfe, wie Kamm, Bürste, Pinsel oder Applicette, und Gebrauchsanleitung enthält. Die Gebrauchsanleitung enthält insbesondere Informationen und Anweisungen für den Verbraucher (m/f) zur Anwendung der Mittel aus den Behältern der Verpackungseinheit in einem Verfahren gemäß dem ersten Erfindungsgegenstand. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht. Das anwendungsbereite Färbemittel wird durch Vermischen der Färbezubereitung (A) mit der Entwicklerzubereitung (B) der Mehrkomponentenverpackungseinheit hergestellt.

Die bevorzugten Ausführungsformen des ersten Erfindungsgegenstands gelten mutatis mutandis für die erfindungsgemäße Verwendung und Verpackungseinheit der weiteren Erfindungsgegenstände. Die nachfolgenden Beispiele sollen bevorzugte Ausführungsformen der Erfindung erläutern, ohne sie jedoch einzuschränken.

1. Beispiele zur Färbung

1.1. Herstellung einer Färbecreme

Aus den aufgelisteten Bestandteilen wurden Blondiercremes wie folgt hergestellt:

| Rohstoff | Gew.-% | |
|---|---|---|
| | E1 | V1 |
| Lanette D | 6,90 | 6,90 |
| Lorol techn. | 2,50 | 2,50 |
| Eumulgin B1 | 0,60 | 0,60 |
| Eumulgin B2 | 0,60 | 0,60 |
| Akypo Soft 45 NV | 10,00 | 10,00 |
| Protelan MST 35 | 6,00 | 6,00 |
| Produkt W37194 | 3,75 | 3,75 |
| 4-Chlorresorcin | 0,01 | 0,01 |
| 2-Methylresorcin | 0,02 | 0,02 |
| 3-Aminophenol | 0,01 | 0,01 |
| 2-Amino-3-hydroxypyridin | 0,01 | 0,01 |
| p-Toluylendiaminsulfat | 0,13 | 0,13 |
| Resorcin | 0,03 | 0,03 |
| Natriumchlorid | 0,62 | 0,62 |
| Natriumsulfit | 0,40 | 0,40 |
| Ascorbinsäure | 0,10 | 0,10 |
| Natriumsilikat 40 / 42 | 0,50 | 0,50 |
| HEDP, 60% | 0,20 | 0,20 |
| KOH-Lösung, 50% | 1,00 | 1,00 |
| Monoethanolamin | 5,00 | 5,00 |
| Glycin | 1,00 | 1,00 |
| Taurin | 1,00 | 1,00 |
| alpha-Liponsäure | 0,20 | 0,20 |
| 4-Acetyl-1-methylpyridinium-p-toluolsulfonat | 1,00 | --- |
| Parfum | qs | qs |
| Wasser | ad 100 | ad 100 |

| | |
|---|---|
| Lanette® D | INCI-Bezeichnung: Cetearyl alcohol; (Cognis) |
| Lorol® tech. | INCI-Bezeichnung: Coconut alcohol (Cognis) |
| Eumulgin® B1 | INCI-Bezeichnung: Ceteareth-12 (Cognis) |
| Eumulgin® B2 | INCI-Bezeichnung: Ceteareth-20 (Cognis) |
| Akypo® Soft 45 NV | INCI-Bezeichnung: Sodium Laureth-5 carboxylate (KAO Chemicals) |
| Protelan® MST 35 | INCI-Bezeichnung: Sodium Myristoyl Sarcosinate, Sodium Methyl Cocoyl Taurate (Zschimmer & Schwartz) |
| Natriumsilikat 40/42 | Natronwasserglas |

Hydrenol D, Lorol, Eumulgin B1, Eumulgin B2 und Akypo Soft 45 NV wurden zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt (pH-Wert 10,64).

Bei der Rezeptur V1 handelt es sich um nicht eine erfindungsgemäße Vergleichsrezeptur, die Rezeptur E1 ist ein erfindungsgemäßes Beispiel mit 4-Acetyl-1-methylpyridinium-p-toluolsulfonat.

1.2. Vermischen mit der Entwicklerdispersion

Die Färbecremes wurden im Verhältnis 1:1 mit einer wie folgt zusammengesetzten Entwicklerdispersion ausgemischt. Der pH-Wert der fertigen Anwendungsmischung lag bei 10,0.

| Rohstoff | Gew.-% |
|---|---|
| Ammoniak, 25 % | 0,62 |
| Dipicolinsäure | 0,10 |
| Dinatriumpyrophosphat | 0,03 |
| Turpinal SL | 1,50 |
| Texapon NSO | 2,00 |
| Dow Corning DB 110 A | 0,07 |
| Aculyn 33 | 12,00 |
| Wasserstoffperoxid 50 % | 22,40 |
| Wasser | ad 100 |

Texapon® NSO ca. 27,5% Aktivsubstanz; INCI-Bezeichnung: Sodium Laureth Sulfate (Cog nis)
Aculyn® 33 ca. 28% Festkörper in Wasser; INCI-Bezeichnung: Acrylates Copolymer (Rohm & Haas)
Dow Corning® DB 110 A INCI-Bezeichnung: Dimethicon (Dow Corning)

Für den Färbeprozess wurde auf Strähnen dunkelblonden und weißen Haares (Codes: Kerling 7/0, EHN) von ca. 0,7 g Gewicht die 4-fache Menge der fertigen Anwendungsmischung appliziert. Nachdem die Strähnen für 30 Minuten bzw. 45 min bei 35 °C behandelt wurden, wurden sie mit einem handelsüblichen Shampoo gewaschen und mit einem Föhn getrocknet.

1.3. Auswertung der Färbeleistung

Die farbmetrischen Messungen erfolgten auf der Strähne an jeweils 4 Messpunkten. Als Messgerät diente der Spectralflash SF 450 der Firma Datacolor.
Die Ergebnisse der Messungen wurden mithilfe des CIELAB Farbenraums quantifiziert.
Der L-Wert steht dabei für die Helligkeit der Färbung (schwarz-weiß-Achse); je größer der Wert für L ist, desto heller ist die Färbung. Der a-Wert steht für die rot-grün-Achse des Systems; je größer der Wert ist, umso mehr ist die Färbung ins Rote verschoben. Der b-Wert steht für die gelb-blau-Achse des Systems; je größer der Wert ist, umso mehr ist die Färbung ins Gelbe verschoben. Farbunterschiede werden mittels des ΔE-Wertes charakterisiert:

$$\Delta E = \sqrt{(L_1^* - L_2^*)^2 + (a_1^* - a_2^*)^2 + (b_1^* - b_2^*)^2}$$

Der Wert für Chromatizität/Farbigkeit errechnet sich nach

$$C = \sqrt{(a)^2 + (b)^2}$$

| Rezeptur | Haartyp | Einwirkzeit [min] | L* | a* | b* | C | ΔE | ΔL | ΔC |
|---|---|---|---|---|---|---|---|---|---|
| V1 | EHN | 30 | 55,1 | 5,8 | 11,8 | 13,1 | 10,2 | 8,6 | 5,6 |
| E1 | EHN | 30 | 63,7 | 8,3 | 16,8 | 18,7 | | | |

(fortgesetzt)

| Rezeptur | Haartyp | Einwirkzeit [min] | L* | a* | b* | C | ΔE | ΔL | ΔC |
|---|---|---|---|---|---|---|---|---|---|
| V1 | EHN | 45 | 55,8 | 5,4 | 11,5 | 12,7 | 0,3 | 7,8 | 6,6 |
| E1 | EHN | 45 | 63,6 | 9,4 | 16,9 | 19,3 | | | |
| V1 | Kerl. 7/0 | 30 | 34,5 | 8,6 | 16,8 | 18,8 | 2,8 | 1,2 | 2,5 |
| E1 | Kerl. 7/0 | 30 | 35,7 | 9,8 | 19,0 | 21,3 | | | |
| V1 | Kerl. 7/0 | 45 | 34,5 | 8,8 | 17,3 | 19,4 | 4,3 | 3,1 | 3,2 |
| E1 | Kerl. 7/0 | 45 | 37,6 | 10,1 | 20,2 | 22,6 | | | |

Die Verwendung des erfindungsgemäßen Färbemittels führte zu einer deutlichen Verbesserung der Farbintensität (ΔE-Wert) der Färbung auf beiden Haartypen.

Ebenso konnte mit dem erfindungsgemäßen Färbemittel eine signifikante Verbesserung der Aufhellleistung (L-Wert) sowie hinsichtlich Farbigkeit C (C-Wert) erreicht werden.

## Patentansprüche

1. Mittel zur oxidativen Färbung keratinischer Fasern, insbesondere menschlicher Haare, enthaltend in einem kosmetischen Träger mindestens eine farbverändernde Komponente, mindestens ein von Luftsauerstoff verschiedenes Oxidationsmittel und mindestens ein Acetylpyridiniumderivat der Formel (I),
worin

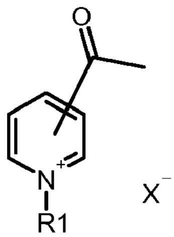

(I),

R1 für eine $C_1$-$C_6$-Alkylgruppe, eine $C_2$-$C_6$-Alkenylgruppe, eine $C_2$-$C_6$-Hydroxyalkyl- gruppe, eine $C_1$-$C_6$-Alkoxy-$C_2$-$C_6$-alkylgruppe, eine Carboxy-$C_2$-$C_6$-alkylgruppe, eine Aryl-$C_1$-$C_6$-alkylgruppe, eine Heteroaryl-$C_1$-$C_6$-alkylgruppe, eine Arylgruppe oder eine Heteroarylgruppe und
$X^-$ für ein physiologisch verträgliches Anion stehen,

**dadurch gekennzeichnet, dass** das Mittel frei von Ammoniak ist.

2. Mittel nach Anspruch 1, **dadurch gekennzeichnet, dass** das Mittel als Acetylpyridiniumderivat 4-Acetyl-1-methylpyridinium-p-toluolsulfonat und/oder 2-Acetyl-1-methylpyridinium-p-toluolsulfonat enthält.

3. Mittel nach einem der Ansprüche 1 und 2, **dadurch gekennzeichnet, dass** das Mittel Acetylpyridiniumderivate gemäß Formel (I) in einer Gesamtmenge von 0,01 bis 5 Gew.-%, bevorzugt von 0,05 bis 3 Gew.-% und insbesondere von 0,1 bis 2 Gew.-%, jeweils bezogen auf das Gesamtgewicht des anwendungsbereiten Färbemittels, enthält.

4. Mittel nach einem der Ansprüche 1 bis 3, **dadurch gekennzeichnet, dass** das Mittel als farbverändernde Komponente mindestens ein Oxidationsfarbstoffvorprodukt und/oder direktziehenden Farbstoff enthält.

5. Mittel nach einem der Ansprüche 1 bis 4, **dadurch gekennzeichnet, dass** das Mittel als von Luftsauerstoff verschiedenes Oxidationsmittel mindestens ein Oxidationsmittel, ausgewählt aus Wasserstoffperoxid und seinen Anlagerungsverbindungen an feste anorganische oder organische Verbindungen, enthält.

6. Mittel nach einem der Ansprüche 1 bis 5, **dadurch gekennzeichnet, dass** das Mittel zusätzlich mindestens eine

Verbindung ausgewählt aus der Gruppe, die gebildet wird aus N-Acylsarcosin-Derivaten der Formel (II) und N-Acyltaurin-Derivaten der Formel (III),

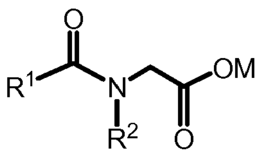 (II), 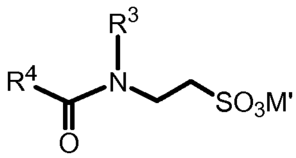 (III),

worin

$R^1$ und $R^4$ jeweils unabhängig voneinander für eine $C_7$-$C_{30}$-Alkylgruppe, $C_7$-$C_{30}$- Alkenylgruppe oder eine $C_7$-$C_{30}$-Hydroxyalkylgruppe stehen,
$R^2$ für eine $C_1$-$C_4$-Alkylgruppe oder eine $C_1$-$C_4$-Hydroxyalkylgruppe steht,
$R^3$ für ein Wasserstoffatom, eine $C_1$-$C_4$-Alkylgruppe oder eine $C_1$-$C_4$- Hydroxyalkylgruppe steht und
M und M' jeweils voneinander unabhängig für ein Wasserstoffatom oder ein Äquivalent eines ein- oder mehrwertigen Kations stehen,

enthält.

7. Mittel nach Anspruch 6, **dadurch gekennzeichnet, dass** das Mittel mindestens eine Verbindung, ausgewählt aus N-Acylsarcosin-Derivaten der Formel (II), und mindestens eine Verbindung, ausgewählt aus N-Acyltaurin-Derivaten der Formel (III), enthält.

8. Mittel nach einem der Ansprüche 1 bis 5, **dadurch gekennzeichnet, dass** das Mittel zusätzlich mindestens eine Verbindung ausgewählt aus der Gruppe, die gebildet wird aus Taurin (2-Aminoethansulfonsäure), Glycin, L-Serin, L-/R-Serin und/oder Coffein, enthält.

9. Kosmetische, nicht therapeutische Verwendung eines Mittels nach einem der Ansprüche 1 bis 8 zur Intensivierung der Färbeleistung bei der oxidativen Färbung keratinischer Fasern, insbesondere menschlicher Haare.

10. Kosmetische, nicht therapeutische Verwendung eines Mittels nach einem der Ansprüche 1 bis 8 zur Verbesserung der Aufhellleistung und/oder Farbigkeit bei der oxidativen Färbung keratinischer Fasern, insbesondere menschlicher Haare.

11. Mehrkomponentenverpackungseinheit (Kit-of-Parts),
umfassend mindestens einem ersten Container (C1) mit einer Färbezubereitung (A), enthaltend in einem kosmetischen Träger

a) mindestens ein Oxidationsfarbstoffvorprodukt und/oder direktziehenden Farbstoff und
b) mindestens ein Acetylpyridiniumderivat der Formel (I),

und mindestens einen zweiten Container (C2) mit einer Entwicklerzubereitung (B), enthaltend in einem kosmetischen Träger mindestens ein Oxidationsmittel,
**dadurch gekennzeichnet, dass** sowohl Färbezubereitung (A) als auch Entwicklerzubereitung (B) frei von Ammoniak sind.

12. Mehrkomponentenverpackungseinheit nach Anspruch 11, **dadurch gekennzeichnet, dass** die Färbezubereitung (A) zusätzlich mindestens eine Verbindung ausgewählt aus der Gruppe, die gebildet wird aus N-Acylsarcosin-Derivaten der Formel (II) und N-Acyltaurin-Derivaten der Formel (III), enthält.

13. Mehrkomponentenverpackungseinheit nach Anspruch 12, **dadurch gekennzeichnet, dass** die Färbezubereitung (A) zusätzlich mindestens eine Verbindung, ausgewählt aus N-Acylsarcosin-Derivaten der Formel (II), und mindestens eine Verbindung, ausgewählt aus N-Acyltaurin-Derivaten der Formel (III), enthält.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10148845 A1
- DE 10261656 A1